(19) Europäisches Patentamt / European Patent Office / Office européen des brevets

(11) **EP 2 794 524 B1**

(12) **FASCICULE DE BREVET EUROPEEN**

(45) Date de publication et mention de la délivrance du brevet:
**05.09.2018 Bulletin 2018/36**

(21) Numéro de dépôt: **12799237.8**

(22) Date de dépôt: **31.10.2012**

(51) Int Cl.:
*C07C 7/13* *(2006.01)*    *C10G 25/05* *(2006.01)*
*B01J 20/18* *(2006.01)*

(86) Numéro de dépôt international:
**PCT/FR2012/000443**

(87) Numéro de publication internationale:
**WO 2013/093219 (27.06.2013 Gazette 2013/26)**

(54) **PROCEDE DE PURIFICATION DE CHARGES OLEFINIQUES A L'AIDE D'UN ADSORBANT COMPRENANT UNE ZEOLITHE DE TYPE 12MR**

VERFAHREN ZUR REINIGUNG VON OLEFINISCHEN ROHSTOFFEN UNTER VERWENDUNG EINES ADSORPTIONMITTELS MIT EINEM ZEOLITH VOM TYP 12MR

PROCESS FOR PURIFYING OLEFINIC FEEDSTOCKS USING AN ADSORBENT COMPRISING A ZEOLITE OF 12MR TYPE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.12.2011  FR 1103997**

(43) Date de publication de la demande:
**29.10.2014  Bulletin 2014/44**

(73) Titulaire: **IFP Energies nouvelles
92500 Rueil-Malmaison (FR)**

(72) Inventeurs:
• **DROZDZ, Sophie**
  **F-69126 Brindas (FR)**
• **BRACCO, Emmanuelle**
  **F-69420 Condrieu (FR)**
• **MARTI, Delphine**
  **F-69003 Lyon (US)**
• **JOLIMAITRE, Eisa**
  **F-69001 Lyon (FR)**

(56) Documents cités:
**FR-A1- 2 903 978    US-A- 3 816 975
US-A- 5 834 392**

**Description**

**Domaine de l'invention:**

L'invention concerne un procédé de purification des hydrocarbures utilisant un adsorbant à réactivité réduite qui présente une teneur en zéolithe très élevée

**Art antérieur:**

Il est connu de l'homme de l'art que les molécules contenant des hétéroatomes (oxygène, soufre, azote etc..) sont néfastes pour le fonctionnement d'un grand nombre de catalyseurs industriels. En effet, ces composés se déposent sur les sites actifs des catalyseurs qui sont alors désactivés et ne sont plus actifs pour catalyser la réaction. En conséquence, de nombreux brevets recommandent d'extraire ces impuretés des coupes avant les réacteurs catalytiques.

La technique la plus efficace pour extraire ces impuretés est l'utilisation d'adsorbants présentant une affinité spécifique pour les impuretés. Le principe de la purification de charges par adsorption est très simple. Le fluide à purifier, par exemple un mélange d'hydrocarbures, est injecté dans une colonne d'adsorption contenant un adsorbant disposé en lit fixe. Dans la colonne, les impuretés sont sélectivement adsorbées dans le solide, ce qui permet de récupérer une coupe purifiée en sortie. Lorsque le solide est saturé, on procède à sa régénération, par circulation d'un désorbant à haute température. Pendant cette phase, la charge est dirigée vers une autre colonne d'adsorption. L'efficacité de ce type de procédé est fortement dépendante des propriétés des solides utilisés. Idéalement, ces solides doivent présenter :

- Une forte capacité d'adsorption pour les impuretés que l'on souhaite extraire.
- Une bonne régénérabilité (la désorption des impuretés doit pouvoir être effectuée dans des conditions de températures raisonnables).
- Une durée de vie la plus élevée possible. Les adsorbants peuvent en effet perdre leur capacité d'adsorption au cours du temps. Ils peuvent par exemple ne pas être suffisamment stables thermiquement et perdre leur propriétés mécaniques au cours des phases de désorption à haute température. La perte en capacité des adsorbants peut également être provoquée par la formation de produits carbonés lourds (coke) au sein de leur porosité, entraînant un bouchage partiel ou total des pores.

La taille des adsorbants utilisés dans les lits fixes est également un paramètre critique. En effet, il est nécessaire que les particules d'adsorbant aient une taille minimale d'environ 0,5 mm afin de limiter les pertes de charge dans le lit.

Concernant la purification des hydrocarbures, les adsorbants de type zéolithiques ont depuis longtemps été identifiés comme des solides présentant de bonnes performances. De part leur polarité, ils présentent une très forte affinité pour les molécules contenant des hétéroatomes. Leur capacité d'adsorption pour ces molécules est donc très élevée, même en présence d'hydrocarbures. Il est en outre possible de les régénérer à des températures de l'ordre de 300°C, ce qui est parfaitement applicable industriellement. Elles présentent enfin un très bonne stabilité thermique.

Il est communément admis que les adsorbants zéolithiques présentent un inconvénient majeur: au cours des cycles successifs d'adsorption et de désorption, des molécules de coke se forment à l'intérieur de leur porosité et leur capacité d'adsorption en est réduite. Ce phénomène est d'autant plus critique que les charges à traiter contiennent des molécules fortement réactives, appelées précurseurs de coke, telles que les hydrocarbures insaturés. Il est en effet bien connu que les molécules insaturées peuvent réagir entre elles pour former des oligomères plus ou moins lourds ainsi que des molécules aromatiques et poly-aromatiques. Ce type de molécules ne peut être extrait de la porosité des zéolithes dans les conditions normales de régénération et sera donc appelé coke dans la suite de ce brevet.

Lorsqu'il s'agit de purifier des coupes contenant des hydrocarbures insaturés, ce problème est bien-sûr particulièrement critique. Ces coupes contiennent des oléfines mono-insaturées et peuvent également contenir des molécules polyinsaturées telles que les dioléfines, qui sont des précurseurs de coke encore plus réactifs que les oléfines mono-insaturées. Par ailleurs, les brevets US 3,816,975, US 6,632,766 US2002/0147377, et US 5,271,835 enseignent que les oléfines co-adsorbées avec les impuretés peuvent s'oligomériser dans les pores des zéolithes, ce qui serait à l'origine de la perte de performance des adsorbants au cours du temps.

En outre, les impuretés elles-mêmes peuvent se comporter de la même manière que des précurseurs de coke. Il est par exemple bien connu que les nitriles sont des groupements très instables et que les mercaptans peuvent réagir avec les molécules oléfiniques pour former des sulfures lourds

Afin de réduire cette perte de capacité d'adsorption par cokage, différentes solutions ont été envisagées.

Certaines propositions concernent des améliorations à apporter au procédé cyclique. Une solution envisagée dans le brevet US 5,271,835 est d'ajouter de l'hydrogène dans le flux de désorbant, à une concentration minimale de 100 ppmv (parties par millions en volume). Cet ajout d'hydrogène permettrait de minimiser les réactions de cokage et donc de maintenir la capacité de l'adsorbant au cours des cycles.

Il est également possible d'éviter au maximum de chauffer l'adsorbant lorsqu'il est en contact avec des molécules insaturées. Ainsi, le brevet US 3,816,975 propose d'insérer une étape supplémentaire au cycle adsorption/désorption classique: après la régénération à haute température par un gaz non adsorbable, le lit est chargé en hydrocarbure saturées en préalable à l'injection de la charge oléfinique. Ainsi, l'augmentation de la température provoquée par l'adsorption des hydrocarbures a lieu en présence de molécules moins réactives. Ce brevet enseigne également que les zéolithes sont responsables des dépôts de coke sur l'adsorbant.

D'autres brevets sont plutôt axés sur une amélioration de la formulation de l'adsorbant, afin de le rendre moins réactif. Ainsi, le brevet US 6,107,535 concerne l'extraction de nitriles d'une coupe hydrocarbonée par adsorption sélective sur des gels de silice. L'intérêt de ce type de matériau est qu'il est très peu acide ce qui permet donc d'éviter la formation de gommes. En revanche, sa capacité d'adsorption pour les nitriles n'est élevé que lorsque la concentration de ces composés dans la charge est de l'ordre de plusieurs milliers de ppm. Or, dans la majorité des cas, la concentration en nitriles dans les coupes oléfiniques est beaucoup plus faible (inférieure à 1000 ppm). La capacité d'adsorption des gels de silice est alors nettement plus faible que celle des zéolithes.

Puisqu'il est communément admis que la formation de coke est due à la trop grande réactivité de la zéolithe, d'autres brevets proposent de réduire la teneur en zéolithe dans l'adsorbant. Les brevets US 5,834,392, US 5,880,052, US 5,858,211 et US 6,019,887 proposent d'utiliser des adsorbants contenant un mélange de zéolithe cationique non acide et d'une matrice inorganique de type oxyde. La zéolithe peut adsorber sélectivement les nitriles alors que la matrice inorganique permet d'adsorber sélectivement et réversiblement les diènes. En outre, la taille des pores de la zéolithe est choisie de manière à laisser entrer les nitrile mais à exclure au maximum l'adsorption des dioléfines. Ainsi, les diènes entrent très peu dans les cages zéolithiques et ne peuvent donc réagir avec les sites actifs des zéolithes. Le solide constitué du mélange de zéolithe et de matrice inorganique est préférentiellement mise en forme à l'aide d'un liant. L'utilisation de ce type de solide présente un inconvénient majeur : la capacité d'adsorption du solide pour le propionitrile est réduite. En effet, ni la matrice organique ni le liant ne présentant d'affinité spécifique pour les nitriles, la masse d'adsorbant nécessaire pour la purification d'une charge donnée est donc accrue.

Le brevet US 6,632,766 propose d'utiliser un adsorbant mis en forme contenant à la fois une zéolithe, un oxyde d'alumine et également un métal, préférentiellement le sodium. L'ajout de sodium permet de réduire la réactivité de l'adsorbant et d'adsorber plus préférentiellement certains composés acides tels que le $CO_2$ et le COS. Cet adsorbant ne contient typiquement que 10 à 60 % de zéolithe, sa capacité d'adsorption pour les impuretés polaires et non acides contenues dans les coupes oléfiniques est donc fortement réduite. Par ailleurs ce brevet, ainsi que le brevet US 5,271,835, enseignent que les zéolites sont responsables de la formation et du dépôt indésirable de coke.

Il apparaît donc que les solutions proposées dans l'art antérieur vont au détriment des capacités d'adsorption des solides utilisés pour les impuretés et que selon l'enseignement de l'art antérieur, les zéolithes sont responsables de le formation de précurseurs de coke et du dépôt de coke.

Nous avons découvert, de manière surprenante, qu'une très forte augmentation de la teneur en zéolithe de type 12MR dans des adsorbants mis en forme permet de réduire la réactivité de ladite zéolithe et donc la formation de coke au cours du temps. L'objet de ce brevet est donc de proposer un procédé utilisant un adsorbant mis en forme à réactivité réduite sans perte de capacité envers les impuretés.

**Résumé de l'invention:**

L'invention concerne un procédé de purification d'une charge d'hydrocarbures comportant des oléfines tel que défini dans la revendication 1.

**Description détaillée de l'invention:**

La présente invention décrit un procédé de purification des hydrocarbures à l'aide d'un adsorbant mis en forme à réactivité réduite vis à vis des molécules insaturées.

Les charges hydrocarbonées concernées par la présente invention contiennent des molécules insaturées. Il peut s'agir de molécules oléfiniques contenant une ou plusieurs double liaisons ou des molécules aromatiques cycliques. En général, le procédé selon l'invention permet de traiter une charge d'hydrocarbures comportant des oléfines (c'est-à-dire des molécules d'hydrocarbures comportant une double liaison entre deux atomes de carbones) et éventuellement des dioléfines (c'est-à-dire des molécules d'hydrocarbures comportant au moins deux double liaison entre deux atomes de carbones). Il n'existe pas de restriction concernant le nombre de carbones des molécules contenues dans ces charges, la seule condition étant que les charges doivent être liquides ou gazeuses à température ambiante, afin de permettre l'écoulement dans les lits d'adsorbants.

La concentration en molécules insaturées des charges selon l'invention peuvent être très diverses. En effet, les molécules insaturées étant plus polaires que les hydrocarbures saturés, elles s'adsorbent préférentiellement dans les adsorbant zéolithiques et peuvent donc provoquer la formation de coke même si elles sont peu concentrées dans la charge.

La charge hydrocarbonées traitées dans le procédé selon l'invention contient une ou plusieurs impuretés à éliminer. Les impuretés contenues dans les charges contiennent au moins un hétéroatome tels que l'oxygène, l'azote et le soufre. Selon la nature de la charge à purifier, la nature et la concentration des impuretés peuvent très fortement varier. Pour illustrer cette variabilité, nous pouvons détailler les impuretés qui sont rencontrées dans différentes charges de la raffinerie. Pour les coupes de type C3-C4 de FCC, les impuretés majoritaires sont généralement l'eau, l'acétonitrile, l'acétone, le méthyl et l'éthylmercaptan, le DMDS et le COS.

Pour les raffinats de MTBE, les impuretés majoritaires sont généralement le méthyl et l'éthylmercaptan, le DMDS et le DEDS, l'acétonitrile, l'acétone, le méthanol, l'éthanol, le MTBE, le TBA et le DME. Pour les coupes C4 de vapocraqueurs, les impuretés majoritaires sont généralement le méthyl et l'éthylmercaptan, le DMDS et le DEDS, l'acétonitrile, le DMF, le NMP, l'acétone. Pour les coupes C5-C6 de FCC, les impuretés majoritaires seront les mercaptans en C1-C3, le thiophène, les nitriles en C2-C4 et le pyrrole.

Les charges des procédés selon l'invention peuvent contenir des teneurs en oléfines polyinsaturées variables. En particulier, il est éventuellement possible de placer le procédé selon l'invention en aval d'un procédé d'hydrogénation sélective, afin de réduire la teneur en oléfines polyinsaturées dans la charge.

L'adsorbant selon l'invention est majoritairement composé de cristaux zéolithiques. La teneur en zéolithe dudit adsorbant est généralement comprise entre 93 %poids et 99,8 %poids, de préférence comprise entre 94 %poids et 99,8 %poids, de manière plus préférée entre 96 %poids et 99,8 %poids, de manière encore plus préférée comprise entre 97 %poids et 99,8 %poids.

La nature de la zéolithe peut varier selon les impuretés à adsorber. Préférentiellement, l'adsorbant selon l'invention comprend une zéolithe dont le diamètres des pores est suffisamment important pour que l'ensemble des impuretés de la charge puisse pénétrer dans le réseau poreux. Le terme diamètre de pore est conventionnel pour l'homme du métier. Il est utilisé pour définir de façon fonctionnelle la taille d'un pore en terme de taille de molécule capable d'entrer dans ce pore. Il ne désigne pas la dimension réelle du pore car celle-ci est souvent difficile à déterminer puisque souvent de forme irrégulière (c'est-à-dire non circulaire). D.W. Breck fournit une discussion sur le diamètre de pore effectif dans son livre intitulé Zeolite Molecular Sieves (John Wiley and Sons, New York, 1974) aux pages 633 à 641. Les sections des canaux des zéolithes étant des anneaux d'atomes d'oxygènes, on peut également définir la taille des pores des zéolithes par le nombre d'atomes d'oxygène formant la section annulaire des anneaux, désigné par le terme « member rings » ou MR en anglais.

Il est par exemple indiqué dans l'« Atlas of Zeolite Structure Types » (W.M. Meier et D.H. Oison, 4ème Edition, 1996) que les zéolithes de type structural FAU ont un réseau de canaux cristallins de 12 MR c'est à dire dont la section est constituée de 12 atomes d'oxygène. Cette définition est bien connue de l'homme de l'art et sera utilisée par la suite. Les zéolithes caractérisées par des diamètres de pores d'au moins 12 MR permettent l'adsorption de nombreuses impuretés et sont donc particulièrement adaptées pour notre application.

Parmi les zéolithes contenant des canaux d'au moins 12 MR, et donc conformes à notre invention, on peut citer les familles suivantes AFI, AFR, BEA, EMT, FAU, LTL, MOR.

Avantageusement, les adsorbants zéolithiques mis en oeuvre dans le procédé contiennent du silicium et au moins un élément T choisi dans le groupe formé par l'aluminium, le bore, le gallium et le fer, de préférence l'aluminium ou le gallium , de manière très préféré l'aluminium.

La teneur en silice de ces adsorbants peut être variable. Le rapport atomique Si/T de la zéolithe selon l'invention est de préférence inférieur à 20, de manière plus préféré inférieur à 15, de manière encore plus préférée inférieur à 8, de manière très préféré inférieur à 6, voir inférieur à 5.

Lorsque l'élément T est l'aluminium, le rapport atomique Si/Al de la zéolite est de préférence inférieur à 8, de manière plus préféré inférieur à 6 et de manière très préféré inférieur à 5, voire inférieur à 4.

La zéolithe contenue dans l'adsorbant selon l'invention est préférentiellement échangées avec des cations d'éléments choisis parmi les éléments alcalins, les éléments alcalino-terreux, les lanthanides ou les métaux de transition. De manière plus préférée lesdits éléments sont choisis parmi les éléments alcalins, les éléments alcalino-terreux et les lanthanides, de manière encore plus préféré parmi les éléments alcalins et les éléments alcalino-terreux, et de manière très préférée parmi les éléments alcalins. Parmi les éléments alcalins, le sodium et le potassium sont préférés et le sodium est l'élément alcalin très préféré. Parmi les éléments alcalino-terreux, le baryum, le magnésium et le calcium sont préférés et le baryum est l'élément alcalino-terreux le plus préféré.

Selon une variante préférée de l'invention, les adsorbants zéolithiques sont choisis parmi la famille des zéolithes de type FAU, qui comprend entre autre les zéolithes suivantes: zéolithe X, zéolithe Y, zéolithe LSX. De manière encore plus préférée, la zéolithe de type FAU selon l'invention est une zéolithe X, Y ou LSX.

Ladite zéolithe de type FAU peut être échangée par tout cation alcalin ou alcalino-terreux. Selon une variante plus préférée, l'adsorbant selon l'invention comprend une zéolithe de type X ou Y ou LSX échangée par du sodium, du potassium ou du baryum. De manière encore plus préférée, l'adsorbant selon l'invention comprend une zéolithe KX, NaX ou BaX. D'une manière très préférée l'adsorbant selon l'invention comprend une zéolithe KX ou NaX, et de manière encore plus préféré un zéolithe NaX.

Pour synthétiser la zéolithe selon l'invention, un gel est préparé en mélangeant, par exemple au moyen d'une turbine, de la silicate de soude, de l'aluminate de sodium et de l'eau. La teneur en chacun des réactifs est ajustée en fonction du ratio atomique Si/Al souhaité. Un mûrissement du gel est opéré pendant un temps variable à une température de préférence proche de 35°C, puis une cristallisation à température plus élevée, de préférence comprise entre 80°C et 200°C, est effectuée. Les cristaux sont ensuite filtrés et lavés.

La taille des cristaux de zéolithe étant de l'ordre de grandeur de quelques micromètres, il est impossible de les utiliser tels-quels dans des adsorbeurs industriels, car les pertes de charge générées seraient beaucoup trop élevées. Il est donc nécessaire de mettre en forme ces cristaux, afin de former des particules de taille plus élevée, c'est à dire de l'ordre du millimètre. Ces particules doivent bien-sûr posséder une résistance mécanique suffisamment élevée, afin de ne pas se détériorer soit au cours des phases de chargement du solide, soit par les pressions exercées par l'écoulement des fluides, ou enfin au cours des étapes de régénération thermique.

Les solides selon l'invention peuvent être mis en forme selon toute technique de mise en forme connue de l'homme de l'art, telle que l'extrusion, la granulation ou le pastillage. Afin d'atteindre des résistances mécaniques compatibles avec une utilisation industrielle, l'ajout d'un liant au cours de l'étape de mise en forme est nécessaire.

En effet, les cristaux de zéolithe ne sont pas suffisamment cohésifs pour pouvoir s'agglomérer en absence de liant, sauf à effectuer du pastillage à très haute pression, technique de mise en forme très onéreuse et susceptible d'entraîner une amorphisation partielle de la zéolithe, et donc une perte en capacité pour l'adsorbant ainsi formé.

Dans le cadre de l'invention, on appelle liant tout matériau ajouté à la zéolithe afin de la mettre en forme. Le taux de liant d'un solide zéolithique mis en forme est donc la teneur massique en tout matériau autre que la zéolithe. La liant et la zéolithe pris en combinaisons représentent donc 100% du solide zéolithique (également appelé adsorbant) mis en forme.

Le liant minéral peut être de préférence choisi parmi les argiles, tels que le kaolin, les minéraux de type palygorskite, tel que l'attapulgite, et les minéraux argileux de type smectite, tels que la montmorillonite ou la bentonite. Le liant peu également être constitué d'oxydes d'alumine ou de silice. Le liant contient préférentiellement au moins 70% d'argile, de préférence ladite argile appartient à la famille des kaolins, kaolinites, nacrite, dickite, halloysites et/ou métakaolins. De manière encore plus préférée, il contient au moins 80% d'argile. Lors de l'étape de mise en forme, des additifs peuvent être ajoutés au mélange de liant et de zéolithe, afin de faciliter l'agglomération ou d'améliorer la résistance mécanique des particules. Les particules issues de la procédure de mise en forme doivent posséder une résistance mécanique suffisante pour pouvoir être utilisées dans un procédé industriel.

La méthode de mise en forme du solide selon l'invention consiste à :

a) synthétiser une zéolithe, selon toute technique connue de l'homme de l'art,

b) mettre en forme cette zéolithe à l'aide d'un liant, avec une teneur en liant de préférence comprise entre 10 et 50% poids. Par exemple on mélange la zéolithe avec le liant et on met en forme le mélange sous forme de grains, par exemple des billes, des extrudés ou tout autre forme.

c) procéder à une zéolithisation des grains obtenus à l'étape b), c'est à dire une transformation du liant en zéolithe, partielle ou totale dudit liant, par toute technique connue de l'homme de l'art. Par exemple, la zéolithisation est effectuée par mise en contact des grains avec une solution basique suivie d'un traitement thermique. La teneur en liant diminue lors de l'étape de zéolithisation, une partie du liant ce transformant en zéolithe.

Cette méthode permet de synthétiser des solides mis en forme avec une très forte teneur en zéolithe et possédant en outre des propriétés mécaniques compatibles avec l'utilisation dans un procédé industriel. Pour évaluer la résistance mécanique des solides, on utilise la méthode de mesure de l'écrasement grain à grain (EGG), qui consiste à mesurer la force de compression maximale que peut supporter un solide avant sa rupture, lorsque le produit est placé entre deux plans se déplaçant à la vitesse constante de 5 cm/min. Lorsqu'il s'agit d'extrudés, donc de solides cylindriques, cette force est rapportée à longueur moyenne des extrudés, et s'exprime donc en daN/mm. S'il s'agit de billes, les valeurs d'EGG sont exprimées directement en daN. La résistance mécanique des grains d'adsorbant obtenu peut être supérieure à 0.4 daN/mm, de préférence supérieure à 0.5 daN/mm, voire 0.6 daN/m pour des extrudés et supérieure à 1.5 daN de préférence supérieure à 2 daN pour des billes.

Lors de l'étape de zéolithisation, le rapport Si/T est susceptible d'être modifié. C'est le rapport Si/T avant zéolitisation qui est pris en compte pour la détermination des caractéristiques de l'adsorbant. Le rapport Si/T revendiqué est donc déterminé avant zéolitisation, et au moyen de toute méthode d'analyse connu de l'homme du métier.

Les adsorbants zéolithiques selon le procédé de l'invention ont une teneur en liant comprise entre 0,2 et 7 %poids, de préférence comprise entre 0,2 et 6%poids, de manière plus préférée comprise centre 0,2 et 4% poids, voire entre 0,2 et 3 %poids de liant. En effet, nous avons découvert que, de manière surprenante, les adsorbants zéolithiques contenant plus de zéolithe 12MR sont non seulement plus capacitifs pour les impuretés des coupes hydrocarbonées, mais également moins réactifs.

Différentes méthodes expérimentales permettant d'estimer la teneur en liant d'un solide mis en forme sont connues de l'homme de l'art. Nous pouvons lister les méthodes suivantes, qui ne sont fournies qu'à titre d'exemple et ne sont pas limitatives :

d) La diffraction des rayons X (DRX). Cette méthode permet d'évaluer la quantité relative de phase amorphe (telle que certains liants) et de phase cristalline (telle que la zéolithe) dans un solide.
e) L'adsorption de différentes molécules. Dans des conditions précises de température et de concentration, on peut mesurer les quantités adsorbées d'une molécule à la fois dans le liant, dans la zéolithe et dans la particule mise en forme. On peut ensuite en déduire la teneur en liant via la formule suivante :

$$x_{liant} = \frac{q_{zéolithe} - q_{particule}}{q_{zéolithe} - q_{liant}}$$

où $x_{liant}$ est la teneur massique en liant, $q_{zéolithe}$ est la concentration en phase adsorbée dans la zéolithe, $q_{particule}$ est la concentration en phase adsorbée dans la particule et $q_{liant}$ est la concentration en phase adsorbée dans le liant. Cette méthode ne peut bien sûr être appliquée que lorsque les concentrations en phase adsorbée sont différentes pour la zéolithe et le liant.

- Une méthode dérivée de la méthode précédente consiste à mesurer les isothermes d'adsorption d'azote à 77K de chacun des trois solides (zéolithe, particule, liant), de calculer les volumes de Dubinin, les volumes microporeux ou les surfaces BET à partir de ces isothermes, et de déterminer la teneur en liant en utilisant la formule :

$$x_{liant} = \frac{P_{zéolithe} - P_{particule}}{P_{zéolithe} - P_{liant}}$$

où $P_i$ correspond à une quelconque valeur caractéristique issue de l'isotherme d'adsorption d'azote (volume de Dubinin, volume microporeux, surface BET,...) pour le solide i. Cette méthode ne peut bien sûr être appliquée que lorsque les valeurs caractéristiques issues de l'isotherme d'adsorption d'azote sont différentes pour la zéolithe et le liant.
- L'analyse de la composition chimique du liant, de la zéolithe (notamment lors de la détermination du rapport Si/T) et de la particule mise en forme, par toute méthode d'analyse connue de l'homme de l'art (analyse par spectrométrie de fluorescence X, par spectrométrie d'absorption atomique). La teneur en liant peut alors être évaluée par la formule : $$x_{liant} = \frac{y_{zéolithe} - y_{particule}}{y_{zéolithe} - y_{liant}}$$ où $y_i$ correspond à la teneur en un élément chimique quelconque présent dans le solide i. Cette méthode ne peut bien sûr être appliquée que lorsque les teneurs en élément chimique sont différentes pour la zéolithe et le liant.

De préférence, le taux de liant des adsorbants selon l'invention est évalué par la méthode du volume de Dubinin, lorsque cela est possible. Cette méthode consiste une estimation du volume microporeux mesuré par adsorption d'azote à 77 K, en supposant que le volume de Dubinin du liant est nul. Le volume de Dubinin décrit ci-dessus est calculé selon la relation de Dubinin-Radushkevich, telle que décrite par Lowell et coll. (Characteηzation of Porous Solids and Powders : Surface Area, Pore Size and Density, chapitre 9, « Micropore Analysis », pages 143-145), et reproduite ci-dessous :

$$\log V = \log V_0 - D \cdot \left( \log \frac{P}{P_0} \right)^2$$

qui relie le volume V d'azote adsorbé dans le matériau adsorbant à la pression relative $P/P_0$. Le volume de Dubinin est le volume $V_0$, volume maximal de vapeur d'azote que l'on peut condenser dans les micropores du matériau adsorbant. Il s'exprime en $cm^3$ de vapeur (azote) (ramenée aux conditions normales) par gramme d'adsorbant.
Préalablement à la mesure, l'échantillon est prétraité à 500°C pendant 12 heures sous vide ($P<5.10^{-6}$ Torr (soit $6,7.10^{-4}$ Pa)). La mesure est ensuite effectuée sur un appareil de type ASAP 2010 M commercialisé par la société Micromeritics. Le gaz adsorbat utilisé est l'azote. Le tracé de l'isotherme est réalisé à l'aide d'une table de pression d'au moins 35 points entre 0,01 et 1 $P/P_0$. On porte sur un diagramme la valeur de log V en fonction de $(\log(P/P_0))^2$. Le volume de Dubinin est obtenu à partir de l'ordonnée à l'origine de la droite de régression linéaire des points dont $(\log(P/P_0))^2$ est compris entre 1 et 2 (soit 0,039 < $P/P_0$< 0,1). L'incertitude de mesure est de ± 0,003.
Selon la composition des charges à traiter, il peut être avantageux d'utiliser l'adsorbant selon l'invention en combinaison avec d'autres adsorbants. Par exemple, lorsque la charge contient de l'eau, il est possible de placer dans la colonne une première couche d'alumine afin d'extraire l'eau, suivie d'une deuxième couche d'adsorbant selon l'invention afin d'extraire les autres impuretés. De même, lorsque la charge contient des molécules azotées et soufrées, les molécules soufrées, qui sont moins sélectivement adsorbées dans les zéolithes, ont tendance à être déplacées par les molécules azotées. Il peut alors être avantageux de placer une couche d'adsorbant sélectif envers les molécules soufrées en amont de la couche d'adsorbant selon l'invention.

Dans le procédé selon l'invention, l'adsorbant peut de préférence être mis en oeuvre en lit fixe. Par exemple après effectué l'étape de mise en contact de l'adsorbant avec la charge, on arrête la mise en contact de l'adsorbant avec la charge et on effectue les étapes suivantes :

i) on régénère l'adsorbant de manière à obtenir un adsorbant appauvri en impureté, puis
ii) on met en contact la masse de captation appauvrie en impureté avec ladite charge.

De préférence à l'étape i), on met en contact l'adsorbant avec un fluide régénérant, le fluide régénérant ayant une température supérieure à la température de la charge ou le fluide régénérant ayant une pression inférieure à la pression de la charge.

Selon une variante préférée, il est possible de mettre en oeuvre l'adsorbant selon l'invention en opérant successivement les étapes suivantes.

a. fournir une charge oléfinique comportant des molécules contenant des hétéroatomes
b. faire circuler la charge dans un lit fixe contenant au moins un adsorbant selon l'invention mis en forme.
c. récupérer la charge purifiée en sortie du lit fixe
d. mettre en contact l'adsorbant avec un fluide régénérant, afin de désorber au moins partiellement les molécules contenant des hétéroatomes

Puis, on répète à nouveau les étapes b à d.

Selon une variante du procédé selon l'invention, l'adsorbant selon l'invention peut être mis en oeuvre selon la technique de modulation de pression (PSA ou Pressure Swing Adsorption selon la technologie anglo-saxonne).

Selon une autre variante préférée du procédé selon l'invention, l'adsorbant est mis en oeuvre selon la technique de modulation de température (TSA ou temperature Swing Adsorption selon la technologie anglo-saxonne). Il est constitué de différentes étapes :

1. Adsorption: la charge est injectée dans le lit d'adsorbant et les impuretés s'adsorbent dans le lit, la charge sort purifiée de la colonne. Cette étape est effectuée à une température comprise entre 15 et 150°C, préférentiellement 20 et 50°C.
2. Déplacement de la charge par le désorbant: arrêt de l'injection de la charge dans la colonne et injection de désorbant. Le désorbant déplace la charge présente dans les volumes morts de la colonne (volumes interstitiels et macro/mésoporeux). Cette étape est effectuée à la même température que l'étape 1.
3. Chauffage du lit d'adsorbant par le désorbant, jusqu'à une température finale comprise entre 200 et 400°C, préférentiellement 250 et 350°C. Pendant cette étape les impuretés sont désorbées de l'adsorbant.
4. Refroidissement: baisse progressive de température dans la colonne par injection de désorbant, jusqu'à atteindre la température d'adsorption.
5. Remplissage de la colonne avec la charge: arrêt de l'injection du désorbant et injection de la charge dans la colonne. La colonne est prête pour une étape d'adsorption.

Ce cycle n'est donné qu'à titre d'exemple et le procédé selon l'invention peut également intégrer des étapes supplémentaires, y compris des étapes intermédiaires localisées entre les étapes 1 à 5.

Selon la composition des charges à traiter, il peut être avantageux d'utiliser le procédé selon l'invention en combinaison avec d'autres techniques de purification. En particulier, il est possible de procéder à un lavage à l'eau de la charge en amont du procédé de purification par adsorption selon l'invention. En effet, certaines impuretés polaires contenues dans les coupes hydrocarbonées peuvent être en grande partie captées dans des colonnes d'extraction à l'eau et il peut être avantageux économiquement de coupler les deux techniques.

**Exemples:**

**Exemple 1 : Synthèse des adsorbants A, B, C et D.**

Cet exemple illustre la réactivité de différents adsorbants composés majoritairement de zéolithe NaX : un adsorbant A selon l'invention et deux adsorbants B et C non conformes à l'invention et représentatifs de l'art antérieur.

**Synthèse de l'adsorbant A (selon l'invention) :**

a) Préparation de la poudre de zéolithe:

On prépare un gel de composition molaire $3,2Na_2O-2,8SiO_2-Al_2O_3-130H_2O$ en utilisant les réactifs suivants : silicate de soude, aluminate de sodium et eau. On laisse le gel mûrir à 35°C pendant 20 heures, puis on opère une cristallisation de 4 heures à 100°C. On filtre et on lave ensuite la poudre contenant des cristaux de zéolithe. Par analyse DRX, on détermine que ces cristaux appartiennent à la famille des faujasite (FAU). L'analyse chimique donne un rapport Si/Al=1,25 +/- 0,03.

b) Agglomération:

Cette poudre de zéolithe est ensuite agglomérée en la mélangeant intimement avec de la kaolinite des Charentes et de la silice colloïdale, avec un ratio massique zéolithe/kaolinite/silice de 12,3/2,3/1, les masses de zéolithe et de kaolinite étant pesées après calcination. Les extrudés sont ensuite séchés et calcinés à 550°C pendant 2 heures.

c) Zéolithisation:

200 g d'extrudés ainsi obtenus sont placées dans un réacteur en verre muni d'une double enveloppe régulée à une température de 100°C, puis on ajoute 1,5 litres d'une solution aqueuse de soude de concentration 100 g/litre et laisse le milieu réactionnel sous agitation pendant 3h. On lave ensuite à l'eau les particules à trois reprises, avant de les activer à 350°C sous azote.

**Synthèse de l'adsorbant B (selon l'art antérieur) :**

a) Préparation de la poudre de zéolithe:

Pour synthétiser la poudre contenant des cristaux de zéolithe de l'adsorbant B, on répète le protocole opératoire de l'étape a) décrit pour l'adsorbant A.

b) Agglomération:

La poudre de zéolithe obtenue à l'étape a) est agglomérée en la mélangeant intimement avec de la kaolinite des Charentes, avec un ratio massique zéolithe/kaolinite de 3/1, les masses de zéolithe et de kaolinite étant pesées après calcination. Les extrudés sont ensuite séchés et calcinés à 550°C pendant 2 heures.
Aucune étape de zéolithisation n'est mise en oeuvre lors de la synthèse de l'adsorbant B.

**Synthèse de l'adsorbant C (selon l'art antérieur) :**

a) Préparation de la poudre de zéolithe:

Pour synthétiser la poudre contenant des cristaux de zéolithe de l'adsorbant B, on répète le protocole opératoire de l'étape a) décrit pour l'adsorbant A.

b) Agglomération:

Cette poudre de zéolithe est ensuite agglomérée en la mélangeant intimement avec de la boehmite, avec un ratio massique zéolithe/boehmite de 4,56/1, les masses de zéolithe et de kaolinite étant pesées après calcination. L'adsorbant C ne contient donc pas de silice. Les extrudés sont ensuite séchés et calcinés à 550°C pendant 2 heures.
Aucune étape de zéolithisation n'est mise en oeuvre lors de la synthèse de l'adsorbant C.

**Synthèse de l'adsorbant D (selon l'art antérieur) :**

a) Préparation de la poudre de zéolithe:

Pour synthétiser la poudre contenant des cristaux de zéolithe de l'adsorbant B, on répète le protocole opératoire de l'étape a) décrit pour l'adsorbant A.

b) Agglomération:

La poudre de zéolithe obtenue à l'étape a) est mélangée avec de la boehmite, avec un ratio massique zéolithe/boehmite de 10,1/1, les masses de zéolithe et de kaolinite étant pesées après calcination. L'agglomération du mélange n'a pas lieu et l'extrusion ne peut donc pas être effectuée. Les solides mis en forme ne peuvent donc pas être synthétisés.
Aucune étape de zéolithisation n'est mise en oeuvre lors de la synthèse de l'adsorbant D.

Le taux de liant des trois adsorbants A,B et C a été évalué par la méthode du volume de Dubinin, qui est une estimation du volume microporeux mesuré par adsorption d'azote à 77 K, en supposant que le volume de Dubinin du liant est nul. Le volume de Dubinin décrit ci-dessus est calculé selon la relation de Dubinin-Radushkevich:

$$\log V = \log V_0 - D \cdot \left( \log \frac{P}{P_0} \right)^2$$

qui relie le volume V d'azote adsorbé dans le matériau adsorbant à la pression relative $P/P_0$. Le volume de Dubinin est le volume $V_0$, volume maximal de vapeur d'azote que l'on peut condenser dans les micropores du matériau adsorbant.
Il s'exprime en $cm^3$ de vapeur (azote) (ramenée aux conditions normales) par gramme d'adsorbant.
Préalablement à la mesure, l'échantillon est prétraité à 500°C pendant 12 heures sous vide (P<5.10$^{-6}$ Torr (soit 6,7.10$^{-4}$ Pa)). La mesure est ensuite effectuée sur un appareil de type ASAP 2010 M commercialisé par la société Micromeritics. Le gaz adsorbat utilisé est l'azote. Le tracé de l'isotherme est réalisé à l'aide d'une table de pression d'au moins 35 points entre 0,01 et 1 $P/P_0$. On porte sur un diagramme la valeur de log V en fonction de $(\log(P/P_0))^2$. Le volume de Dubinin est obtenu à partir de l'ordonnée à l'origine de la droite de régression linéaire des points dont $(\log(P/P_0))^2$ est compris entre 1 et 2 (soit 0,039 < $P/P_0$< 0,1).

Les teneurs en liant obtenues pour les trois échantillons sont précisées dans le tableau 1.

**Tableau 1 : teneur en liant et EGG des adsorbants de type NaX**

|  | Teneur en liant (% massique) | EGG (daN/mm) |
|---|---|---|
| Adsorbant A (selon l'invention) | 5+/-2 | 0,9 |
| Adsorbant B (selon l'art antérieur) | 24+/-3 | 0,8 |
| Adsorbant C (selon l'art antérieur) | 19+/-3 | 0,8 |

Sur le tableau 1, on peut également noter que les trois adsorbants possèdent des EGG supérieurs à 0,6 et donc compatibles avec une utilisation dans des adsorbeurs industriels.

La réactivité des trois adsorbants A,B et C a ensuite été testée comme suit. Chaque adsorbant a été activé sous azote à 400°C pendant 2 heures. 100 mg d'adsorbant est ensuite transféré dans une fiole (de type vial selon le terme anglo-saxon) contenant 250 ml de pentadiène. Le solide est maintenu en contact avec le pentadiène pendant 15 heures. Ensuite, environ 30 mg de solide est prélevé de ladite fiole et placé dans la nacelle d'une thermobalance SETARAM sous un flux d'hélium de 3 Nl/h.

Une programmation de température est ensuite appliquée dans la balance et, en parallèle, la perte en masse de l'échantillon est mesurée. Dans un premier temps, la température est maintenue à 30°C pendant 60 minutes, afin d'évaporer le pentadiène condensé dans la macroporosité. L'échantillon est ensuite chauffé jusqu'à 500°C avec une rampe de 5°C/minute, puis maintenu à 500°C pendant 30 minutes. En maintenant toujours cette même température, on injecte alors dans la thermobalance, un gaz composé à moitié d'hélium et à moitié d'air, afin de bruler le carbone résiduel, et ce pendant une durée de 2 heures.
Pendant toute la durée de l'expérience, l'effluent gazeux en sortie de thermobalance est analysé par un spectromètre de masse, ce qui permet de différencier la perte de masse provoquée par la désorption du pentadiène de celle provoquée par la désorption ou le brulage du coke. On évalue finalement la réactivité du solide en calculant le pourcentage de masse de coke par rapport à la masse totale désorbée.
Les résultats obtenus sont consignés dans le tableau 2 ci-dessous.

**Tableau 2: comparaison de la réactivité des adsorbants A, B et C**

|  | % massique de pentadiène désorbé | % masse de coke désorbé ou brulé |
|---|---|---|
| Adsorbant A (selon l'invention) | 73 | 27 |
| Adsorbant B (selon l'art antérieur) | 64 | 36 |
| Adsorbant C (selon l'art antérieur) | 57 | 43 |

Les résultats du tableau 2 montrent qu'au contact avec du pentadiène, le solide selon l'invention forme nettement moins de coke que les solides selon l'art antérieur.

### Exemple 2 : Capacité d'adsorption des adsorbants A, B et C

Cet exemple permet de comparer les capacités d'adsorption des mêmes échantillons que ceux définis dans l'exemple 1.

Pour ce faire, les échantillons sont activés sous azote à 400°C pendant 2 heures. Il sont ensuite mis en contact dans une fiole (de type vial selon le terme anglo-saxon) avec une solution d'héxène contenant différentes concentrations d'acétonitrile. La fiole est ensuite placée dans un bain vibrant et thermostaté à 30°C pendant 24 heures. La teneur en acétonitrile finale est ensuite analysée par chromatographie, ce qui permet de calculer par bilan matière la quantité d'acétonitrile adsorbée dans chacun des adsorbants à différentes concentrations.

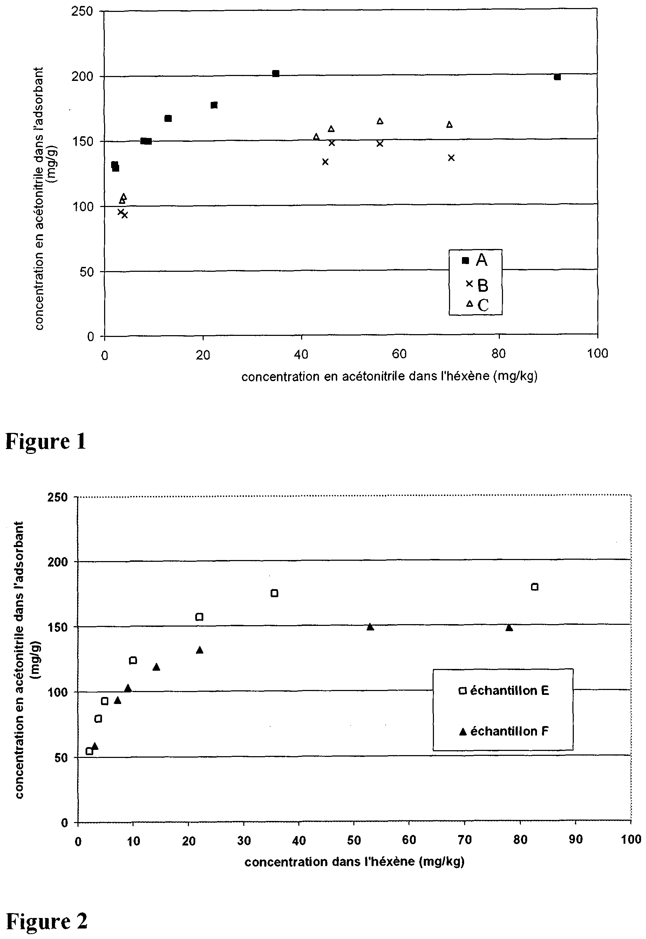

Les résultats obtenus sont présentés sur la figure 1.

La Figure 1 montre que la capacité d'adsorption d'acétonitrile par l'adsorbant A selon l'invention est supérieure à celles des adsorbants B et C selon l'art antérieur. L'adsorbant A selon l'invention présente donc une capacité d'adsorption plus élevée tout en formant moins de coke.

### Exemple 3 : Synthèse des adsorbants E et F

Cet exemple montre que la réactivité de différents adsorbants composés majoritairement de zéolithe KX : un adsorbant selon l'invention et un adsorbant conforme à l'art antérieur.

**Synthèse de l'adsorbant E selon l'invention :**

a) Préparation de la poudre de zéolithe:

On prépare un gel de composition molaire 3,2 $Na_2O$ - 2,8 $SiO_2$ - $Al_2O_3$-130 $H_2O$ en utilisant les réactifs suivants : silicate de soude, aluminate de sodium et eau. On laisse le gel mûrir à 35°C pendant 20 heures, puis on opère une cristallisation de 4 heures à 100°C. On filtre et on lave ensuite les cristaux. Par analyse DRX, on détermine que ces cristaux appartiennent à la famille des faujasite (FAU). L'analyse chimique donne un rapport Si/Al=1,25 +/- 0,03.

b) Agglomération:

Cette poudre de zéolithe est ensuite agglomérée en la mélangeant intimement avec de la kaolinite des Charentes et de la silice colloïdale, avec un ratio massique zéolithe/kaolinite/silice de 12,3/2,3/1, les masses de zéolithe et de kaolinite étant pesées après calcination. Les extrudés sont ensuite séchés et calcinés à 550°C pendant 2 heures.

200 g de particules ainsi obtenues sont placées dans un réacteur en verre muni d'une double enveloppe régulée à une température de 100°C, puis on ajoute 1,5 litres d'une solution aqueuse de soude de concentration 100 g/l et laisse le milieu réactionnel sous agitation pendant 3h. On lave ensuite à l'eau les particules à trois reprises, avant de les activer à 350°C sous azote.

Echange cationique:

On met ensuite les extrudés en contact avec une solution de KCl à 1,2N pendant 1heure dans un rotavapor régulée à une température de 80°C. Le solide est ensuite récupéré puis rincé abondamment avec de l'eau distillée jusqu'à ce que le pH de la solution de rinçage soit équivalent à celui de l'eau distillée. Enfin, le solide est séché en étuve à 100°C durant une nuit. On réitère à trois reprises la procédure de mise en contact avec le KCl afin de s'assurer de l'échange total des cations.

**Synthèse de l'adsorbant F (selon l'art antérieur) :**

a) Préparation de la poudre de zéolithe:

On commence par synthétiser des cristaux de zéolithe NaX, selon le même protocole opératoire que pour l'adsorbant A selon l'invention.

Echange cationique:

On procède ensuite à l'échange cationique selon la procédure décrite pour l'adsorbant E ci-avant

b)Agglomération:

Cette poudre de zéolithe est ensuite agglomérée en la mélangeant intimement avec de la kaolinite des Charentes, avec un ratio massique zéolithe/kaolinite de 5,25/1, les masses de zéolithe et de kaolinite étant pesées après calcination. Les extrudés sont ensuite séchés et calcinés à 550°C pendant 2 heures.
Les teneurs en liant obtenues pour les trois échantillons selon la méthode des volumes de Dubinin (cf. exemple 1) sont précisées dans le tableau 3.

**Tableau 3 : teneur en liant des adsorbants de type KX**

|  | Teneur en liant (% massique) | EGG (daN/mm) |
|---|---|---|
| Adsorbant E (selon l'invention) | 3+/-2 | 0.9 |
| Adsorbant F (selon l'art antérieur) | 16+/-3 | 0.8 |

Sur le tableau 3, on peut également noter que les deux adsorbants E et F possèdent des EGG supérieurs à 0,6 et donc compatibles avec une utilisation dans des adsorbeurs industriels.
Les réactivités des deux solides sont ensuite testées selon la méthodologie explicitée dans l'exemple 1. Les résultats obtenus sont consignés dans le tableau 4 ci-dessous.

**Tableau 4: comparaison de la réactivité des adsorbants E et F**

|  | % massique de pentadiène désorbé | % masse de coke désorbé ou brulé |
|---|---|---|
| Adsorbant E (selon l'invention) | 77 | 23 |
| Adsorbant F (selon l'art antérieur) | 68 | 32 |

Le résultats consignés dans le Tableau 4 montrent que l'adsorbant selon l'invention permet de former moins de coke que l'adsorbant selon l'art antérieur.

**Exemple 4 : Capacité d'adsorption des adsorbants E et F**

Cet exemple permet de comparer les capacités d'adsorption des échantillons synthétisés dans l'exemple 3.
La procédure expérimentale est identique à celle décrite dans l'exemple 2.
Les résultats obtenus sont présentés sur la figure 2.
La Figure 2 montre que la capacité d'adsorption d'acétonitrile par l'adsorbant selon l'invention est supérieure à celles des adsorbants conformes à l'art antérieur. L'adsorbant E selon l'invention présente donc une capacité d'adsorption plus élevée tout en formant moins de coke.

**Exemple 5** : Synthèse de l'adsorbant G (selon l'art antérieur)

Cet exemple illustre la nécessité d'ajouter un liant pour conférer aux particules mises en forme une résistance mécanique suffisante.

a) Préparation de la poudre de zéolithe:

On commence par synthétiser des cristaux de zéolithe NaX, selon le même protocole opératoire que pour l'adsorbant A selon l'invention.

b) Mise en forme:

Le pastillage des cristaux de zéolithe NaX est réalisé a l'aide d'une presse de marque Korsch (modele EKO). Le poinçon inférieur présentant un trou de diamètre 3,5 mm permet de mettre en forme les cristaux de zéolithe sous forme de pastilles de 3,5 mm de diamètre. Le sabot de remplissage de la presse est rempli de cristaux de NaX et on procède au compactage avec les paramètres suivants: filière de 15 mm de profondeur, profondeur de pénétration du poinçon égale à 11 mm, vitesse de rotation du rotor égale à 25 tours par minute, dimensions de chaque pastille: diamètre égal a 3,5 mm, épaisseur égale a 4 mm.

A la fin de la procédure de pastillage, la pastille de NaX est très friable et se délite au moment de son extraction du sabot de la presse. La poudre de NaX ne peut donc être mise en forme sans liant par pastillage, la résistance mécanique de la particule ainsi formée est insuffisante.

## Revendications

1. Procédé de purification d'une charge d'hydrocarbures comportant des oléfines et au moins une impuretés comportant au moins un hétéroatome, dans lequel on effectue une étape de mise en contact de la charge avec un adsorbant comprenant entre 93% poids et 99,8%poids de zéolithe et entre 0,2 et 7 %poids de liant après l'étape c) de zéolithisation, ledit liant étant choisi parmi les argiles, les minéraux de type palygorskite, et les minéraux argileux de type smectite, la zéolithe étant de type 12MR, dans lequel ladite zéolithe contient du silicium et un élément T choisi dans le groupe constitué par l'aluminium, le bore, le gallium et le Fer, et dans lequel le rapport atomique Si/T de la zéolithe est inférieur à 20, ledit rapport Si/T étant déterminé avant zéolithisation, lequel adsorbant ayant été obtenu en effectuant les étapes suivantes :

    a) on synthétise ladite zéolithe,
    b) on mélange ladite zéolithe avec ledit liant et on met en forme le mélange sous forme de grains, ledit mélange comportant entre 10 % et 50% poids de liant,
    c) on effectue une zéolithisation des grains obtenus à l'étape b).

2. Procédé de purification selon la revendication 1, dans lequel le rapport atomique Si/T de la zéolithe est inférieur à 15.

3. Procédé de purification selon l'une des revendications 1 et 2 dans lequel ledit élément T est l'aluminium ou le gallium.

4. Procédé de purification selon l'une des revendications 1 et 2 dans lequel l'élément T est l'aluminium.

5. Procédé de purification selon la revendication 4 dans lequel le rapport atomique Si/Al de la zéolithe est inférieur à 8.

6. Procédé de purification selon l'une des revendications 1 à 5 dans lequel ladite zéolite est échangée avec des cations d'éléments choisis parmi les éléments alcalins, les éléments alcalino-terreux, les lanthanides ou les métaux de transition.

7. Procédé de purification selon l'une des revendications 1 à 6 dans lequel ladite zéolite est choisie dans le groupe constitué par les zéolithes AFI, AFR, BEA, EMT, FAU, LTL, MOR.

8. Procédé de purification selon l'une des revendications 1 à 7 dans lequel ladite zéolite est une zéolite KX ou NaX.

9. Procédé selon la revendication 8 dans lequel ladite zéolite est une zéolite NaX.

10. Procédé de purification selon l'une des revendications 1 à 9 dans lequel ledit liant contient au moins 70% d'argile.

11. Procédé de purification selon l'une des revendication 1 à 10, dans lequel l'adsorbant est sous forme d'extrudé et a une résistance mécanique est supérieure à 0.4 daN/mm, la résistance mécanique étant déterminée par la méthode de mesure de l'écrasement grain à grain (EGG)

12. Procédé de purification selon l'une des revendications 1 à 10, dans lequel l'adsorbant est sous forme de bille et a une résistance mécanique supérieure à 1.5 daN, la résistance mécanique étant déterminée par la méthode de mesure de l'écrasement grain à grain (EGG).

13. Procédé de purification selon l'une des revendications 1 à 12 dans lequel après l'étape de mise en contact,

   i) on régénère l'adsorbant de manière à obtenir un adsorbant appauvri en impureté, puis
   ii) on met en contact la masse de captation appauvrie en impureté avec ladite charge.

14. Procédé de purification selon la revendication 13, dans lequel à l'étape i), on met en contact l'adsorbant avec un fluide régénérant, le fluide régénérant ayant une température supérieure à la température de la charge ou le fluide régénérant ayant une pression inférieure à la pression de la charge.


**Patentansprüche**

1. Verfahren zur Reinigung eines Kohlenwasserstoffrohstoffs, umfassend Olefine und mindestens eine Vereinreinigung, umfassend mindestens ein Heteroatom, wobei ein Schritt zum Inkontaktbringen des Rohstoffs mit einem Adsorptionsmittel, umfassend zwischen 93 Gew.-% und 99,8 Gew.-% Zeolith und zwischen 0,2 und 7 Gew.-% Bindemittel, nach dem Schritt c) zum Zeolithisieren durchgeführt wird, wobei das Bindemittel ausgewählt ist aus Tonen, Mineralien vom Typ Palygorskit und Tonmineralien vom Typ Smektit, wobei der Zeolith vom Typ 12MR ist, wobei der Zeolith Silizium und ein Element T enthält, ausgewählt aus der Gruppe bestehend aus Aluminium, Bor, Gallium und Eisen, und wobei das Si/T-Atomverhältnis des Zeolithen kleiner 20 ist, wobei das Si/T-Verhältnis vor dem Zeolithisieren bestimmt wird, wobei das Adsorptionsmittel durch Ausführen der folgenden Schritte erhalten wurde:

   a) Synthetisieren eines Zeolithen,
   b) Mischen des Zeolithen mit dem Bindemittel und Formen der Mischung in Pelletform, wobei die Mischung zwischen 10 Gew.-% und 50 Gew.-% Bindemittel umfasst,
   c) Ausführen einer Zeolithisierung der in Schritt b) erhaltenen Pellets.

2. Reinigungsverfahren nach Anspruch 1, wobei das Si/T-Atomverhältnis des Zeolithen kleiner 15 ist.

3. Reinigungsverfahren nach einem der Ansprüche 1 und 2, wobei das Element T Aluminium oder Gallium ist.

4. Reinigungsverfahren nach einem der Ansprüche 1 und 2, wobei das Element T Aluminium ist.

5. Reinigungsverfahren nach Anspruch 4, wobei das Si/Al-Atomverhältnis des Zeolithen kleiner 8 ist.

6. Reinigungsverfahren nach einem der Ansprüche 1 bis 5, wobei der Zeolith mit Kationen von Elementen, ausgewählt aus alkalischen Elementen, erdalkalischen Elementen, Lanthaniden oder Übergangsmetallen, ausgetauscht ist.

7. Reinigungsverfahren nach einem der Ansprüche 1 bis 6, wobei der Zeolith ausgewählt ist aus der Gruppe bestehend aus den Zeolithen AFI, AFR, BEA, EMT, FAU, LTL, MOR.

8. Reinigungsverfahren nach einem der Ansprüche 1 bis 7, wobei der Zeolith ein Zeolith KX oder NaX ist.

9. Verfahren nach Anspruch 8, wobei der Zeolith ein Zeolith NaX ist.

10. Reinigungsverfahren nach einem der Ansprüche 1 bis 9, wobei das Bindemittel mindestens 70 % Ton enthält.

11. Reinigungsverfahren nach einem der Ansprüche 1 bis 10, wobei das Adsorptionsmittel in extrudierter Form vorliegt und eine mechanische Festigkeit von mehr als 0,4 daN/mm aufweist, wobei die mechanische Festigkeit durch das Messen der Druckfestigkeit einzelner Pellets (Single Pellet Crush Strength (SPCS)) bestimmt wird.

12. Reinigungsverfahren nach einem der Ansprüche 1 bis 10, wobei das Adsorptionsmittel in Kugelform vorliegt und eine mechanische Festigkeit von mehr als 1,5 daN aufweist, wobei die mechanische Festigkeit durch das Verfahren zum Messen der Druckfestigkeit einzelner Pellets (Single Pellet Crush Strength (SPCS)) bestimmt wird.

13. Reinigungsverfahren nach einem der Ansprüche 1 bis 12, wobei nach dem Schritt zum Inkontaktbringen

i) das Adsorptionsmittel regeneriert wird, um ein verunreinigungsarmes Adsorptionsmittel zu erhalten, dann
ii) die verunreinigungsarme Einfangmasse mit dem Rohstoff in Kontakt gebracht wird.

14. Reinigungsverfahren nach Anspruch 13, wobei in Schritt i) das Adsorptionsmittel mit einem Regenerationsfluid in Kontakt gebracht wird, wobei das Regenerationsfluid eine Temperatur aufweist, die über der Temperatur des Rohstoffs liegt, oder das Regenerationsfluid einen Druck aufweist, der niedriger ist als der Druck des Rohstoffs.

**Claims**

1. Process for purification of a hydrocarbon feedstock comprising olefins and at least one impurity comprising at least one heteoratom, in which a stage for bringing the feedstock into contact with an adsorbent comprising between 93% by weight and 99.8% by weight of zeolite and between 0.2 and 7% by weight of binder is carried out after the step c) of zeolithization, said binder being chosen among clay, palygorskite-type minerals, and smectite-type clay minerals, with the zeolite being of the 12 MR type, in which said zeolite contains silicon and an element T selected from the group that consists of aluminum, boron, gallium and iron, and in which the Si/T atomic ratio of the zeolite is less than 20, said ratio Si/T being determined before zeolithisation, said adsorbent being obtained by carrying out the following stages:

   a) A zeolite is synthesized,
   b) The zeolite is mixed with a binder, and the mixture is shaped in the form of grains, with the mixture comprising between 10% and 50% by weight of binder,
   c) A zeolithization of grains obtained in stage b) is carried out.

2. Process for purification according to Claim 1, in which the Si/T atomic ratio of the zeolite is less than 15.

3. Process for purification according to one of Claims 1 and 2, in which said element T is aluminum or gallium.

4. Process for purification according to one of Claims 1 and 2, in which the element T is aluminum.

5. Process for purification according to Claim 4, in which the Si/Al atomic ratio of the zeolite is less than 8.

6. Process for purification according to one of Claims 1 to 5, in which said zeolite is exchanged with cations of elements selected from among the alkaline elements, the alkaline-earth elements, the lanthanides, or the transition metals.

7. Process for purification according to one of Claims 1 to 6, in which said zeolite is selected from the group that consists of the zeolites AFI, AFR, BEA, EMT, FAU, LTL, and MOR.

8. Process for purification according to one of Claims 1 to 7, in which said zeolite is a KX or NaX zeolite.

9. Process according to Claim 8, in which said zeolite is an NaX zeolite.

10. Process for purification according to one of Claims 1 to 9, in which said binder contains at least 70% clay.

11. Process for purification according to one of Claims 1 to 10, in which the adsorbent is in the form of an extrudate and has a mechanical strength that is greater than 0.4 daN/mm, with the mechanical strength being determined by the method for measuring grain-to-grain crushing (EGG).

12. Process for purification according to one of Claims 1 to 10, in which the adsorbent is in the form of a ball and has a mechanical strength that is greater than 1.5 daN, with the mechanical strength being determined by the method for measuring grain-to-grain crushing (EGG).

13. Process for purification according to one of Claims 1 to 12, in which after the contact stage,

   i) The adsorbent is regenerated in such a way as to obtain an adsorbent that is low in impurities, and then
   ii) The collection mass that is low in impurities is brought into contact with said feedstock.

14. Process for purification according to Claim 13, in which in stage i), the adsorbent is brought into contact with a regenerating fluid, the regenerating fluid having a temperature that is higher than the temperature of the feedstock or the regenerating fluid having a pressure that is lower than the pressure of the feedstock.

Figure 1

Figure 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 3816975 A
- US 6632766 B
- US 20020147377 A
- US 5271835 A
- US 6107535 A
- US 5834392 A
- US 5880052 A
- US 5858211 A
- US 6019887 A

**Littérature non-brevet citée dans la description**

- le diamètre de pore effectif. **D.W. BRECK.** Zeolite Molecular Sieves. John Wiley and Sons, 1974, 633-641
- **W.M. MEIER ; D.H. OISON.** Atlas of Zeolite Structure Types. 1996
- Micropore Analysis. **LOWELL.** Characterization of Porous Solids and Powders : Surface Area, Pore Size and Density. 143-145